(19) **Europäisches Patentamt / European Patent Office / Office européen des brevets**

(11) **EP 4 682 686 B1**

(12) **EUROPEAN PATENT SPECIFICATION**

(45) Date of publication and mention
of the grant of the patent:
**20.05.2026  Bulletin 2026/21**

(21) Application number: **25189356.6**

(22) Date of filing: **14.07.2025**

(51) International Patent Classification (IPC):
**G06F 3/01** (2006.01)      **G06N 3/09** (2023.01)
**A61B 5/00** (2006.01)      **G06N 3/045** (2023.01)
**G06N 3/0455** (2023.01)    **G06N 3/0464** (2023.01)
**G06N 3/047** (2023.01)     **G06N 3/08** (2023.01)

(52) Cooperative Patent Classification (CPC):
**G06F 3/015; A61B 5/7267; G06F 3/017;
G06N 3/045; G06N 3/0455; G06N 3/0464;
G06N 3/047; G06N 3/08; G06N 3/09**

(54) **METHOD, DEVICE, AND PROGRAM OF RESTORING TRAJECTORY OF HAND MOVEMENT BASED ON BIOSIGNAL**

VERFAHREN, VORRICHTUNG UND PROGRAMM ZUR WIEDERHERSTELLUNG DER BEWEGUNGSBAHN EINER HANDBEWEGUNG AUF DER BASIS VON BIOSIGNALEN

PROCÉDÉ, DISPOSITIF ET PROGRAMME DE RESTAURATION DE TRAJECTOIRE DE MOUVEMENT DE MAIN SUR LA BASE D'UN BIOSIGNAL

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **15.07.2024  KR 20240093287**

(43) Date of publication of application:
**21.01.2026  Bulletin 2026/04**

(73) Proprietor: **Korea University Research and Business Foundation**
**Seoul 02841 (KR)**

(72) Inventors:
• **LEE, Seong Whan**
  **06292 Seoul (KR)**
• **KIM, Jun Young**
  **10129 Gimpo-si, Gyeonggi-do (KR)**
• **LEE, Seo Hyun**
  **06007 Seoul (KR)**

(74) Representative: **BCKIP Part mbB**
**MK1**
**Landsbergerstraße 98, 3.Stock**
**80339 München (DE)**

(56) References cited:
  **US-A1- 2014 058 528      US-A1- 2021 018 896**

• ZHANG WEIMING ET AL: "An Approach for Upper Limb Movement Intention Recognition Using EEG and sEMG Fusion based on the MCPSA-CIIM", 2023 WRC SYMPOSIUM ON ADVANCED ROBOTICS AND AUTOMATION (WRC SARA), IEEE, 19 August 2023 (2023-08-19), pages 402 - 407, XP034432618, DOI: 10.1109/ WRCSARA60131.2023.10261809

• AL-QURAISHI MAGED S ET AL: "Multimodal Fusion Approach Based on EEG and EMG Signals for Lower Limb Movement Recognition", IEEE SENSORS JOURNAL, IEEE, vol. 21, no. 24, 11 October 2021 (2021-10-11), pages 27640 - 27650, XP011893244, ISSN: 1530-437X, [retrieved on 20211214], DOI: 10.1109/JSEN.2021.3119074

• BALASUBRAMANIAN SIVAKUMAR ET AL: "Is EMG a Viable Alternative to BCI for Detecting Movement Intention in Severe Stroke?", IEEE TRANSACTIONS ON BIOMEDICAL ENGINEERING, IEEE, USA, vol. 65, no. 12, 1 December 2018 (2018-12-01), pages 2790 - 2797, XP011697416, ISSN: 0018-9294, [retrieved on 20181120], DOI: 10.1109/TBME.2018.2817688

• HO JIN CHOI ET AL: "On the Feasibility of EEG-based Motor Intention Detection for Real-Time Robot Assistive Control", ARXIV.ORG, CORNELL UNIVERSITY LIBRARY, 201 OLIN LIBRARY CORNELL UNIVERSITY ITHACA, NY 14853, 13 March 2024 (2024-03-13), XP091698944

**Description**

**BACKGROUND OF THE INVENTION**

**Field of the Invention**

The present invention relates to a method and device of training an artificial neural network for inferring a trajectory of an intended hand movement based on a biosignal. More specifically, the present invention relates to a method and device of providing a user's intended hand movement or character as a trajectory or text based on a biosignal when there is a hand movement such as handwriting so as to allow various interface interactions such as communication and drawing.

**Background of the Related Art**

A biosignal includes electroencephalography (EEG), electromyography (EMG), and the like.

Electroencephalography is a noninvasive technique that measures an electrical activity in the brain. For example, brain waves may be captured through electrodes attached to the skull, and shown as a graph of voltage change over time. Electroencephalography is widely used to diagnose and study various brain-related diseases, and plays an important role in neuroscience, psychology, and medical research in particular. Basically, electroencephalography measures signals originating in different regions of the brain. Those signals are mainly caused by the electrical activity of neurons, and can be analyzed because they have different characteristics in terms of region, time, and frequency. Electroencephalography is an important biosignal, especially because it contains fundamental information on a hand movement and includes a user's implicit intention. Compared to another representative biosignal such as fMRI, electroencephalography has a fast temporal resolution, and can detect brain activity in minute time units, but the signal-to-noise ratio is high due to various obstacles such as the scalp, skull, and hair between the electrodes. Additionally, the spatial resolution is relatively low, so it is often difficult to determine exactly where in the brain the signal originates. Therefore, the quality of technology using only electroencephalography is affected by appropriate noise removal and characteristic extraction techniques.

Electromyography is a technology that measures and analyzes the electrical activity of muscles. The technology captures electromyography through electrodes attached to the muscles, allowing the detailed movements and intentions of the muscles to be identified. The technology is widely applied in medicine, rehabilitation, and sports science as it is used to monitor muscle contraction and relaxation. Electromyography signals are based on the electrical activity of muscle fibers. The signals originate from action potentials generated in motor nerve fibers when a muscle is activated, and may be used to quantitatively measure muscle strength, endurance, and fatigue. The temporal and spatial resolutions of electromyography are generally higher than those of electroencephalography. The signal-to-noise ratio of electromyography varies depending on the quality of contact between the muscle and the electrode, external noise, and signal processing method, and the like, and noise removal and appropriate characteristic extraction may be required.

Proprioception is the sense of recognizing a position and movement of the body, and may be obtained through proprioceptors distributed in muscles, tendons, joints, and skin. Proprioception tells us how our body parts (such as hand joints) are positioned in space and how they are moving.

Deep learning is a branch of machine learning that focuses on data modeling using artificial neural networks, and may solve difficult problems through complex interactions of multiple layers of neural networks. Deep learning shows high performance in high-dimensional and unstructured data such as biosignals, and is utilized in various application fields such as image recognition, natural language processing, and voice recognition.

Artificial intelligence models in the field of deep learning may have a structure of an encoder and a decoder. The encoder may nonlinearly extract complex characteristics from input data to form a 'latent representation'. The latent representation is a concise representation of the intrinsic properties or structures of the data, a transformation of the original data into a simpler, more processable form. The decoder may utilize the latent representation to generate or predict the desired outcome. The decoder may perform tasks such as classification, regression, image reconstruction, text translation, and speech recognition, for example. The term 'latent space' refers to an abstract space in which all possible latent representations exist, where various characteristics of the data are mathematically represented. In various embodiments of the present invention, the latent representation may also be referred to as a 'latent vector' or an 'embedding'.

Taking image reconstruction as an example, an image encoder analyzes a given image to transform important information into a compressed form, that is, a latent representation. For example, when an encoder processes an image of a cat, it recognizes the characteristics of the cat such as its size, position, breed, facial expression, and contours, and compresses them into a concise data form. Such a latent representation contains core characteristics of a cat image. An image decoder may use latent representations to reconstruct new images that resemble the original image, generate different poses or facial expressions of the cat, or classify images. The latent space holds a 'core element' of the data, and small changes within the latent space may be largely attributed to changes in the characteristics of the image. For example, a shift in a specific direction in a latent space may change a cat's facial expression, and a shift in a different direction may result in a change in color or background. As such, latent representations help effectively process complex data such as images, and have a wide range of applications such as creative image creation, efficient image compression, and sophisticated image editing.

The encoder is automatically trained using latent representations in a direction of minimizing a difference (loss value) between a result output by the decoder and measured data. Through this, the latent space may be automatically optimized according to a structure and objective function of a model to be structured into a more accurate and efficient latent space. The latent space may be formed by automatic learning, or may also be artificially adjusted based on a result of human data modeling. For example, latent representations may be adjusted to emphasize or ignore specific data characteristics, and specific patterns or characteristics may be enhanced through separate data reconstruction models. Adjustment of latent representations in a latent space may be achieved through several technical methods. For example, a mechanism such as a 'concatenation', 'sum', or 'attention' may be used to combine or enhance different latent representations.

The encoder/decoder structure, which is a concept that can be generally applied to most artificial intelligence models, may provide a foundation that helps artificial intelligence models effectively process complex data, and allow for more diverse and creative results to be derived through flexible manipulation of the latent space.

A brain-computer interface (BCI), which is a technology that can directly transmit a user's intentions or thoughts to a computer system, is a technology that connects the brain and a computer to recognize the user's intentions and perform various control tasks using only imagination. BCI technology is being actively researched in various fields, especially in medicine, games, and virtual reality. The BCI technology is being used to provide functions such as message transmission, environmental control, and voice synthesis, especially for people with limitations in movement or communication.

In the existing BCI-based user interface control method, instruction input using brain waves was common. For example, methods such as steady-state visual evoked potentials (SSVEP) were used to allow a user to analyze brain waves in response to visual stimuli of a specific frequency, thereby performing cursor movement, character selection, and simple instruction execution. The method, which can simplify and execute complex control commands, is also used as an assistive device for patients who have difficulty in communication.

Camera-based tracking, motion capture using various sensors, and touch screens are utilized to track the movement of a human body, for example, handwriting/hand tracking. Camera-based tracking captures and analyzes the movement of a human body in real time using external or built-in cameras. Motion capture uses sensors to measure the movement of a human body.

Document KR 10-2021-0059079 A discloses a hand tracking system using depth camera and electromyogram sensors.

Document KR 10-2020-0110998 A discloses a virtual reality interaction hand tracking system using multiple sensors and implementation method thereof.

Document KR 10-2023-0086936 A discloses a VR apparatus and method for simulating writing reality in metaverse and VR environments through interworking writing equipment.

Document KR 10-2013-0141904 A discloses a half-field SSVEP (Steady State Visually Evoked Potentials) based BCI (Brain Computer Interface) system and motion method thereof.

Document "An approach for upper limb movement intention recognition using EEG (electroencephalography) and sEMG (surface electromyography) fusion based on the MCPSA-CIIM (Multi-scale Convolution, Polarized Self-Attention, and Cross Intelligence Integration Module)", Zhang Weiming, 2023 WRC Symposium on Advanced Robotics and Automation, IEEE, pages 402-407, retrieved on 2023-09-27, discloses recognising upper-limb movement intention recognition using neural networks (NN), trained on EEG and EMG signals.

Document "Multimodal fusion approach based on EEG (electroencephalography) and EMG (electromyography) signals for lower limb movement recognition", Al-Quraish Maged S., IEEE Sensors Journal, vol. 21, no. 24, retrieved on 2021-12-14, discloses machine-learning (ML) classification based on fusion of EEG and EMG data to detect lower limb movement intention.

Document US 2021 / 0 018896 A1 discloses a system and method comprising a noninvasive framework utilizing electroencephalography (EEG) to achieve the neural control of a robotic device for continuous random target tracking.

Document US 2014 / 0 058 528 A1 discloses a noninvasive brain computer interface (BCI) system including an electroencephalography (EEG) electrode array configured to acquire EEG signals generated by a subject. The subject observes movement of a stimulus. A computer is coupled to the EEG electrode array and configured to collected and process the acquired EEG signals. A decoding algorithm is used that analyzes low-frequency (delta band) brain waves in the time domain to continuously decode neural activity associated with the observed movement.

Document "Is EMG a Viable Alternative to BCI for Detecting Movement Intention in Severe Stroke?", Balasubramanian Sivakumar, IEEE Transactions on Biomedical engineering, vol. 65, no. 12, retrieved on 2018-11-20, discloses movement intention recognition with a NN trained EMG signals, instead of EEG signals, which are regarded as too noisy.

Document "On the feasibility of EEG-based motor intention for real-time robot assistive control", Ho Jin Choi, ARXIV.ORG, Cornell university library, 201 Olin library Cornell university Ithaca, NY 14853, submitted on 13 March 2024, discloses detection of user motion intention with a support vector machine (SVM) classifier trained on filtered EEG signals.

## SUMMARY OF THE INVENTION

Existing user interface devices that track the movement of a hand or handwriting are provided with cameras, sensors, and/or touch devices, and have problems such as a spatial constraint, a sensor dead space, and a limitation in capturing a fine movement caused by installing those external devices at a predetermined distance from a user. In addition, existing BCI-based communication devices use external stimuli, which requires a high level of concentration and training, so it is often difficult to execute complex instructions. Tracking movement using only electroencephalography among biometric signals is difficult to expect various interactions with users because only simple control can be decoded due to the high signal-to-noise ratio of electroencephalography. There is a growing need for a method and device that can overcome those limitations and be used more flexibly and user convenience in various environments and situations.

According to the present disclosure, only the user's biosignal are used for movement tracking so that there is no spatial constraint and square space due to the use of a camera, and by applying electroencephalography containing the user's intrinsic intention, and output a hand movement such as handwriting intended (imagined) by the user even if the user does not actually move the hand by applying electroencephalography containing the user's intrinsic intention. In addition, a user-interface device can be controlled in various ways purely by the user by utilizing a deep learning model trained by synthesizing various biometric information. That is, a user's intrinsic biosignal is decoded without external stimulation, thereby providing user convenience and allowing restoration of complex hand movements such as handwriting trajectories rather than simple classification.

Meanwhile, technical problems are not limited to the above-mentioned problems, and other technical problems which are not mentioned herein will be clearly understood by those skilled in the art from the description below.

Although electroencephalography provides fast temporal resolution, its low signal-to-noise ratio makes it difficult to track direct hand movement. To complement electroencephalography, proprioceptive data may be utilized to infer more accurate movement paths as well as imagined hand movements. This allows for more accurate detection of the user's actual or intended movements, which in turn allows for more sophisticated user-interface interactions. Proprioceptive data may be obtained by measuring and then modeling acceleration, angular velocity, and electromyography (EMG) of the hand joints. Such modeling may be achieved by a deep learning reconstruction-based model. Accelerometers and angular velocity sensors monitor joint movements in real time, which allows precise tracking of hand position and movement path. Electromyography may measure muscle contraction and relaxation through the electrical activity of muscles so as to detect even minute movements of the hand. In particular, proprioceptive information may be an important intermediate step in inferring a clean movement path (trajectory) from noisy electroencephalography (EEG) .

Accordingly, a model is trained based on actual hand movement data such that an encoder extracts proprioceptive latent information from EEG, and a decoder generates a movement path based on the proprioceptive latent information. The method allows robust decoding even for an imagined hand movement. It has been scientifically proven that imagined movements show brain activity patterns similar to those seen in actual movements. Because the brain processes imagined movements in the same way as real movements and activates similar neural pathways, information such as proprioception may be embedded in electroencephalography even in those imagined movements, and composite models based on proprioceptive information and electroencephalography may operate effectively while maintaining high accuracy and reliability even under various environmental factors. This approach is advantageous in processing noisy EEG signals and predicting imagined movement paths, and in conclusion, the utilization of such proprioceptive information is an important background technology, which may contribute to improving the accuracy and efficiency of user-interface interactions based on various biosignals.

To this end, the present invention provides a method of training an artificial neural network for inferring a trajectory of an intended hand movement based on a biosignal in accordance with claim 1 and a device of training an artificial neural network for inferring a trajectory of an intended hand movement based on a biosignal in accordance with claim 9. According to the present invention, the method includes collecting, by a first data collection part, electroencephalography data and electromyography data related to a user's hand movement; collecting, by a second data collection part, acceleration data and angular velocity data related to the user's hand movement; performing, by a first preprocessing part, preprocessing on each of the collected electroencephalography data, electromyography data, acceleration data, and angular velocity data; extracting, by a proprioception data extraction part, proprioception data based on the preprocessed electromyography data, acceleration data, and angular velocity data; and training, by a data learning part, trajectory data of the user's hand movement through an artificial neural network based on the preprocessed electroencephalography data and the extracted proprioception data.

The training of trajectory data of the user's hand movement may include extracting proprioceptive latent representations used for data reconstruction of electromyography data, angular velocity data, and acceleration data from the extracted proprioception data; extracting a first free latent representation used for trajectory tracking and a first joint latent representation used for proprioceptive tracking from the preprocessed electroencephalography data; and determining a predicted trajectory based on the extracted proprioceptive latent representation, the first free latent representation, and the first joint latent representation. The data learning part may determine the predicted trajectory by using a weighted integration for each of: a reconstruction loss value indicating a similarity between data reconstructed by the data reconstruction and actual measurement data; a joint latent representation loss value indicating a similarity between a first joint latent representation and a proprioceptive latent representation; and a trajectory loss value indicating a similarity between the predicted trajectory and an actual trajectory, as a loss function. Each of the weights may vary over time.

The preprocessing on the collected electroencephalography data and electromyography data may include filtering by a band-stop filter, filtering by a band-pass filter, independent component analysis to remove unnecessary signals, and filtering by a low-frequency band-pass filter, and the preprocessing of the angular velocity data and acceleration data may include filtering by a correction filter.

The method may further include, subsequent to performing the training of the data learning part, collecting, by a third data collection part, electroencephalography data of a target related to hand movement imagination of the target; performing, by a second preprocessing part, preprocessing on the collected electroencephalography data of the target; and inferring, by a data inference part, trajectory data of a hand movement of the target through the artificial neural network based on the preprocessed electroencephalography data of the target. The inferring of the trajectory data of the hand movement of the target may further include extracting, by an electroencephalography encoder, a second free latent representation used for trajectory tracking and a second joint latent representation used for proprioceptive tracking based on the preprocessed electroencephalography data input through the artificial neural network; and inferring, by a trajectory decoder, a trajectory of hand movement of the target based on the second free latent representation and the second joint latent representation. A trajectory data processing part may control to transmit or display trajectory data of the inferred hand movement of the target.

Advantages of the present invention comprise providing a device and method capable of performing user-interface interactions by recognizing an inherent intention of a target user even in a state of minute movement or no movement.

Advantagess of the present invention also comprise not simply classifying the movements of a target user, but rather inferring the handwriting/hand movements intended by the user without spatial constraints and sensor blind spots in a manner of restoring the user's intended hand movement from biosignals, thereby allowing user-interface control with complexity such as handwriting, drawing, and moving a cursor. This allows a user to easily interact with a mobile phone or other mobile electronic device by writing, drawing, or manipulating using only his or her imagination even in situations where it is difficult to use hands, such as physically disabled people with uncomfortable arms or rush hour subways, driving, and injuries.

## BRIEF DESCRIPTION OF THE DRAWINGS

FIG. 1 is a block diagram of a device that restores a trajectory of hand movement according to one embodiment.
FIG. 2 is a flowchart of an operation performed by a device that restores a trajectory of hand movement according to one embodiment
FIG. 3 is a block diagram of a process for learning and inferring a user's movement using a biosignal according to one embodiment.
FIG. 4 shows a process of a data preprocessing module according to one embodiment.
FIG. 5 shows a process of a learning module according to one embodiment.
FIG. 6 shows a process of an inference module according to one embodiment.

## DETAILED DESCRIPTION OF THE PREFERRED EMBODIMENT

The details of the objects and technical configurations of the present invention and operational effects thereof will be more clearly understood from the following detailed description based on the accompanying drawings appended hereto. Hereinafter, embodiments of to the present invention will be described in detail with reference to the accompanying drawings.

Embodiments disclosed herein should not be interpreted as limiting or used to limit the scope of the present invention. It is apparent for those skilled in the art that a description including embodiments herein has various applications. Therefore, any embodiments described in the detailed description of the present invention are illustrative for better understanding of the present invention and are not intended to limit the scope of the present invention to the embodiments.

The invention is defined by the appended independent claims, with relevant embodiments according to the dependent claims.

Functional blocks illustrated in the drawings and described hereunder are only examples of possible implementations. In other implementations, other functional blocks may be used without departing from the concept and scope of the detailed description. Furthermore, one or more functional blocks are illustrated as separate blocks, but one or more of the functional blocks may be a combination of various hardware and software elements that execute the same function.

In addition, an expression that some elements are "included" is an expression of an "open type", and the expression simply denotes that the corresponding elements are present, but should not be construed as excluding additional elements.

Moreover, in case where it is mentioned that one element is "connected" or "coupled" to the other element, it should be understood that one element may be directly connected to the other element, but another element may be present therebetween.

Hereinafter, various embodiments of the present invention will be described with reference to the accompanying drawings. However, it should be understood that the embodiments are not intended to limit the present invention to specific embodiments.

In various embodiments of the present invention, a user may be used interchangeably with a subject. In various embodiments of the present invention, a target may be used interchangeably with a target user or a target subject. In various embodiments of the present invention, a user and a target may be separate or may overlap at least in part.

In various embodiments of the present invention, hand movements are illustrated, but are not limited thereto, and the present invention may also be applied to movements of other body parts of a user or target.

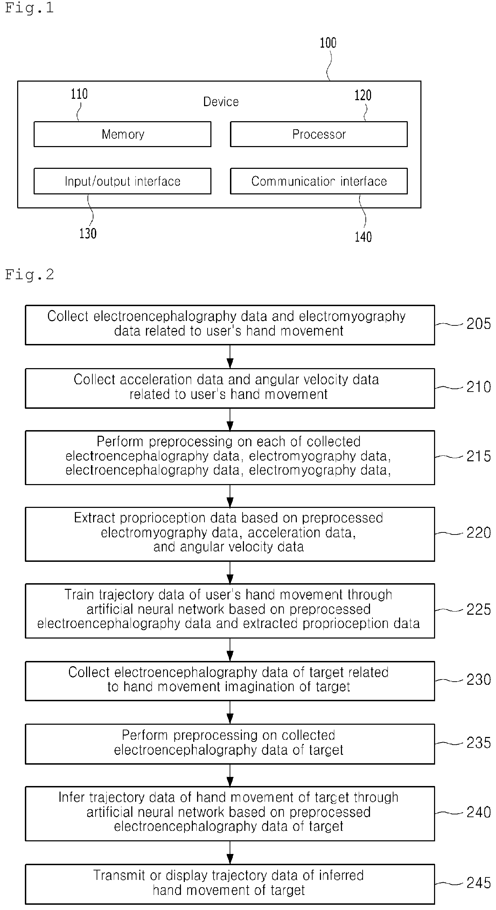

FIG. 1 is a configuration diagram of a device 100 that restores a trajectory of hand movement (hereinafter, referred to as a 'device 100') according to one embodiment.

Referring to FIG. 1, the device 100 according to one embodiment may each include a memory 110, a processor 120, an input/output interface 130, and a communication interface 140.

The memory 110 may store data acquired from an external device or data generated by itself. The memory 110 may store instructions that can perform an operation of the processor 120. For example, the memory 110 may store collected data, preprocessed data, extracted data, an artificial neural network, and the like.

The processor 120 is an operational device that controls an overall operation. The processor 120 may execute instructions stored in the memory 110. The operation of the device 100 according to an embodiment of the invention may be understood as an operation performed by the processor 120.

The input/output interface 130 may include a hardware interface or software interface that inputs and outputs information.

The communication interface 140 allows information to be transmitted and received through a communication network. To this end, the communication interface 140 may include a wireless communication module or a wired communication module.

The device 100 may be implemented as various types of devices capable of performing operations through the processor 120 and transmitting and receiving information through a network. For example, it may be implemented in a form of a server, a computer device, a portable communication device, a smart phone, a portable multimedia device, a laptop, a tablet PC, and the like, but is not limited to those examples.

The elements of the device 100 may be divided in a different manner than in FIG. 1. According to another aspect, the device 100 is defined to include a first data collection part, a second data collection part, a first preprocessing part, a proprioception data extraction part, and a data learning part. The device 100 may be defined further to include a third data collection part, a second preprocessing part, a data inference part, and a trajectory data processing part.

FIG. 2 is a flowchart of an operation performed by the device 100 according to one embodiment. The operation of the device 100 according to an embodiment in FIG. 2 may be understood as an operation performed by the processor 120.

Each step disclosed in FIG. 2 is only a preferred embodiment in achieving the objectives of the present invention, and some steps may be added thereto or deleted therefrom as needed, and any one step may be included in another step to be performed. The order of respective operations disclosed in FIG. 2 is only arranged for convenience of understanding, and such an order is not limited to a time series order, and the order may be changed and operated differently depending on the designer's choice.

In step 205, a first data collection part collects electroencephalography data and electromyography data related to a user's hand movement. The collected data may be stored in a predetermined database.

In step 210, a second data collection part collects acceleration data and angular velocity data related to the user's hand movement. The collected data may be stored in a predetermined database. When using a biosignal with high deviation for each individual, it may be difficult to use a large amount of data, and thus auxiliary data such as acceleration data and angular velocity data may be utilized to increase consistency in deep learning even with a small amount of data.

In step 215, a first preprocessing part performs preprocessing on each of the collected electroencephalography data, electromyography data, acceleration data, and angular velocity data. The preprocessing on the collected electroencephalography data and electromyography data may include filtering by a band-stop filter, filtering by a band-pass filter, independent component analysis to remove unnecessary signals, and filtering by a low-frequency band-pass filter. The preprocessing of the angular velocity data and acceleration data may include filtering by a correction filter.

In step 220, a proprioception data extraction part extracts proprioception data based on the preprocessed electromyography data, acceleration data, and angular velocity data.

In step 225, a data learning part trains trajectory data of the user's hand movement through an artificial neural network based on the preprocessed electroencephalography data and the extracted proprioception data. The training of the trajectory data of the user's hand movement may include extracting a proprioceptive latent representation used for data reconstruction of electromyography data, angular velocity data, and acceleration data from the extracted proprioception data, extracting a first free latent representation used for trajectory tracking and a first joint latent representation used for proprioceptive tracking from the preprocessed electroencephalography data, and determining a predicted trajectory based on the extracted proprioceptive latent representation, the first free latent representation, and the first joint latent representation.

The data learning part may determine the predicted trajectory by using a weighted integration for each of: a reconstruction loss value indicating a similarity between data reconstructed by the data reconstruction and actual measurement data; a joint latent representation loss value indicating a similarity between a first joint latent representation and a proprioceptive latent representation; and a trajectory loss value indicating a similarity between the predicted trajectory and an actual trajectory, as a loss function. Each of the weights may be configured to vary over time.

In step 230, a third data collection part may collect electroencephalography data of a target related to hand movement imagination of the target. The target may be separate from the user, or may overlap at least in part.

In step 235, a second preprocessing part may perform preprocessing on the collected electroencephalography data of the target. The preprocessing on the collected electroencephalography data of the target may include filtering by a band-stop filter, filtering by a band-pass filter, independent component analysis to remove unnecessary signals, and filtering by a low-frequency band-pass filter.

In step 240, a data inference part may infer trajectory data of a hand movement of the target through the artificial neural network based on the preprocessed electroencephalography data of the target. The inferring of the trajectory data of the hand movement of the target may include extracting, by an electroencephalography encoder, a second free latent representation used for trajectory tracking and a second joint latent representation used for proprioceptive tracking based on the preprocessed electroencephalography data input through the artificial neural network, and inferring, by a trajectory decoder, a trajectory of hand movement of the target based on the second free latent representation and the second joint latent representation.

In step 245, a trajectory data processing part may control to transmit or display trajectory data of the inferred hand movement of the target. A device that receives the transmitted trajectory data may store or display the trajectory data.

A human operation process goes through a simplified series of steps: (1) Collecting data from sensory organs, (2) analyzing sensory data in the brain and establishing motor plans, (3) transmitting action commands to muscle nerves, and (4) performing actual actions through muscle contraction and relaxation. Since brain waves generated during such an operation process are noisy and difficult to decode, a deep learning model must be able to efficiently simulate the above process in order to restore detailed movements such as high-dimensional handwriting based on the brain waves. To this end, 'proprioception' data is collected, which is directly related to a hand movement and is obviously processed in the brain, and guide the model to learn such a proprioceptive latent representation, thereby improving efficiency. Proprioception includes a position of a joint, a tension of a muscle, a speed and direction of movement, and the brain establishes and executes an accurate motor plan based on such information. When a deep learning model simulates such a human operation process, the utilization of proprioception data may reduce the complexity of brain waves, and build a model that is robust to noise. Specifically, proprioception data is used as auxiliary information to help interpret electroencephalography signals, thereby allowing the model to act as a link between noisy electroencephalography signals and precise hand movement trajectories. This approach may significantly improve the accuracy of electroencephalography-based motor decoding.

According to various embodiments of the present invention, data such as a user's electroencephalography, electromyography, joint-specific angular velocity and acceleration may be collected, and an encoder/decoder model may be trained to embody latent representations in consideration of proprioceptive information, so as to allow to operate, in actual use, using only electroencephalography, and to restore a corresponding hand movement simply by imagining a hand movement.

According to various embodiments of the present invention, an electroencephalography encoder may correspond to a neural encoder, and may comprehensively include deep learning layers such as a fully connected layer, a convolutional neural network layer, a long short-term memory, a recurrent neural network, and a transformer to nonlinearly extract features from a biosignal so as to create a useful embedding.

According to various embodiments of the present invention, a trajectory decoder may infer an embedding of a neural encoder as a trajectory, and may comprehensively include deep learning layers such as a fully connected layer, a convolutional neural network layer, a long short-term memory, a recurrent neural network, and a transformer to infer an actual trajectory on a useful composite embedding consisting of a free latent representation and a proprioceptive latent representation created from a neural encoder.

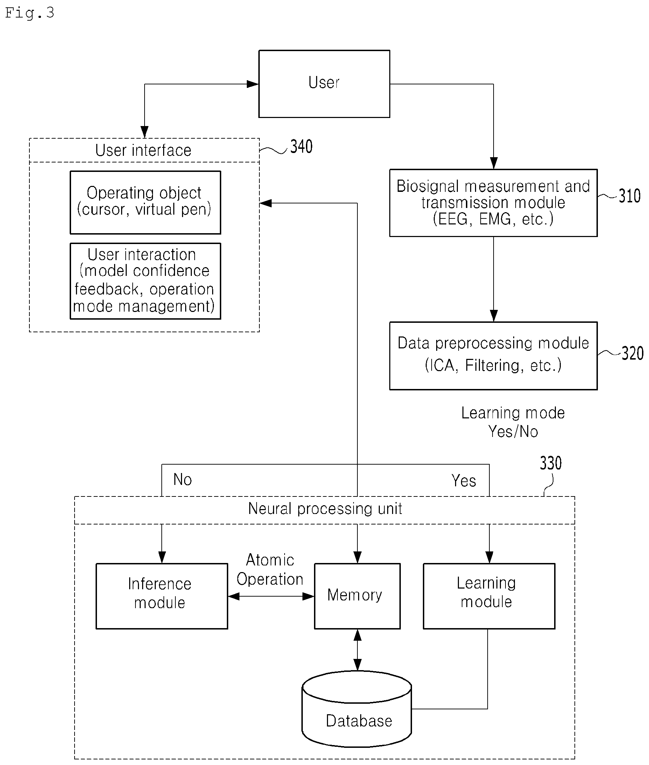

FIG. 3 is a block diagram of a process for learning and inferring a user's movement using a biosignal according to one embodiment.

A biosignal measurement and transmission module 310 collects biosignals (electroencephalography and electromyography) during a user's intended hand movement (including both actual and imagined movements). A data preprocessing module 320 performs noise removal on the collected biosignals through independent component analysis, and filtering by a band-stop filter, and/or a band filter. A neural processing unit 330 infers the user's intended hand movement trajectory based on the preprocessed biosignal, which may be output to a display by a user interface 340. The neural processing unit 330 may be mounted with a learning module for maintaining continuous performance in a user-customized manner and an inference module for inferring the user's intention in real time. In various embodiments of the present invention, the neural processing unit 330 performs learning that takes proprioception into account by using electromyography, acceleration, and/or angular velocity signals as auxiliary information in addition to electroencephalography during learning, and may use only electroencephalography among biosignals during inference.

In various embodiments of the present invention, a trajectory restoration process of hand movement may be divided into two modes: a learning mode and an inference mode. The learning mode is a process of learning a deep neural network through the learning module based on the user's arm movements and their biosignals according to a set learning scenario, and updating the inference module by fine-tuning the deep neural network through transfer learning to maintain steady performance in the inference mode. The inference mode, which may also be called an operation mode, is a process of continuously outputting the user's intentional arm movements through the inference module using the deep neural network model obtained through the learning mode.

The biosignal measurement and transmission module 310 may be divided into a biosignal collection module and a transmission module. The biosignal collection module is a module that collects biosignals by recording a voltage difference of an electrode sensor. The biosignal collection module consists of a device using noninvasive electrodes, which may be attached to the scalp and arm, for example, to collect signals (electromyography and electroencephalography) at a sampling frequency (e.g., 1000 Hz). In various embodiments of the present invention, acceleration signals and angular velocity signals based on an inertial measurement unit (IMU) sensor attached to a body part (e.g., a joint) of a user or target may be additionally collected. A (voltage) signal collected from the biosignal collection module may be input to the data preprocessing module through the transmission module.

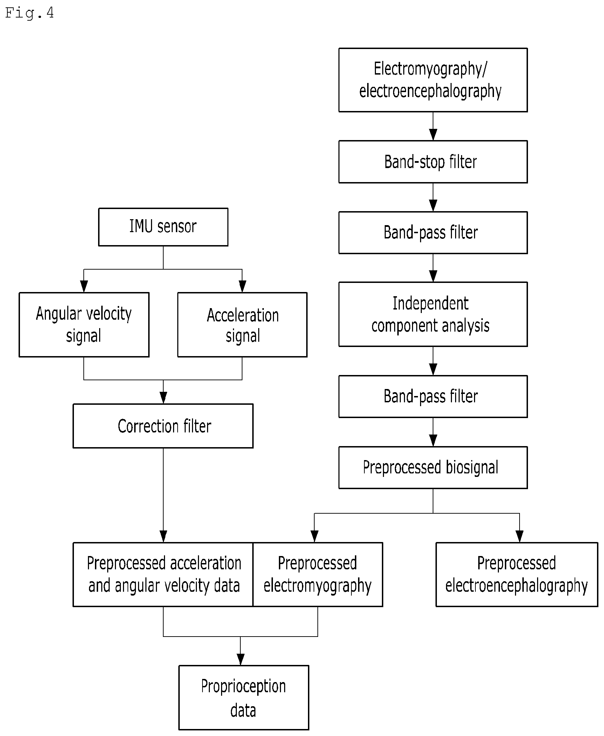

FIG. 4 shows a process of a data preprocessing module according to one embodiment.

The data preprocessing module 320 is a module that preprocesses biosignals (electromyography/electroencephalography) transmitted from the biosignal collection module to remove noise. In an example of FIG. 4, preprocessing of electromyography and electroencephalography involves three frequency filtering steps and one dimension reduction step. Specifically, first, a signal of a predetermined frequency (e.g., 60 Hz) and a noise signal corresponding to its harmonics (e.g., 120 Hz, 180 Hz, etc.) coming from a transmission line in a surrounding environment are removed through a band-stop filter. Then, white noise and other high frequency noises are removed through a band (pass) filter (1 to 45 Hz). Independent component analysis is performed with noise primarily removed through a band filter to remove noise signals generated by other movements such as eye blinking. Then, a biosignal frequency band optimized for a hand movement is extracted through a band (pass) filter (1 to 8 Hz).

Although not shown in FIG. 4, electromyography and electroencephalography signals may be further optimized for learning or inference by additional preprocessing such as baseline correction, windowing, and normalization.

Acceleration and angular velocity signals output from the IMU sensor may be used as proprioception data by integrating them with preprocessed electromyography after passing through a correction filter such as a Kalman filter or a complementary filter.

The neural processing unit 330 may include a learning module and an inference module, and may be additionally configured with a database and a memory for an intermediate process of updating the inference module using a learning result and outputting an inference result and feedback from the device. In the learning module, a deep neural network to be described later is fine-tuned by using preprocessed biosignals and trajectory data. The fine-tuned deep neural network is stored in the database and transmitted to the inference module through the memory. The inference module is always running, and the learning module may be turned on/off according to the user's needs. While the learning module is on, the user may perform a hand movement according to a predetermined scenario, and the inference model may also be updated in real time.

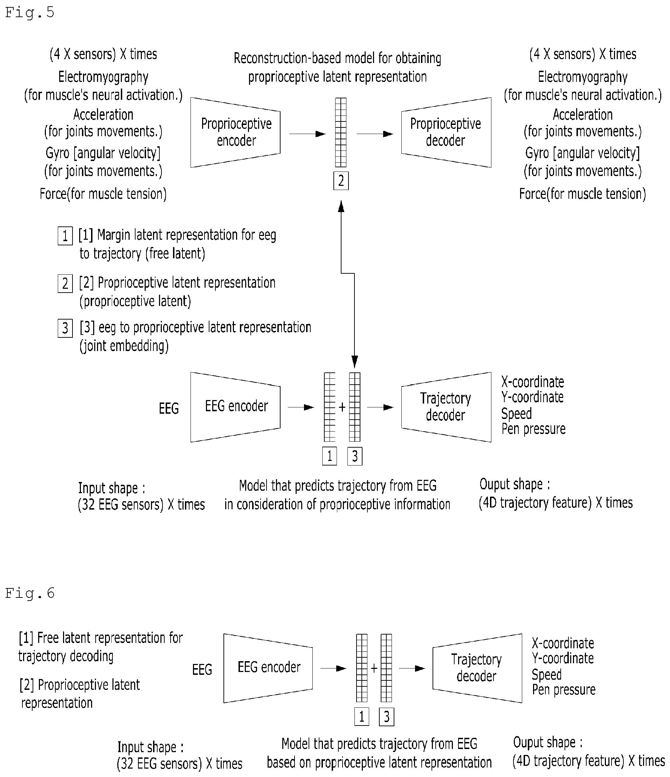

FIG. 5 shows a process of a learning module according to one embodiment.

A learning module may consist of a proprioceptive reconstruction unit (a reconstruction-based model for obtaining a proprioceptive latent representation) and a main trajectory inference unit (a model that predicts a trajectory from electroencephalography (EEG) in consideration of proprioceptive information). The proprioceptive reconstruction unit is a reconstruction-based model designed to reconstruct a user's proprioceptive information and aims to create an optimal latent representation for proprioception data. The main trajectory inference unit aims to infer (predict) a hand movement trajectory from EEG in consideration of proprioceptive information. The main trajectory inference unit may consist of an EEG encoder and a trajectory decoder. The EEG encoder may be trained to extract features by considering both proprioceptive latent representations from EEG and latent representations containing important features for trajectory inference. The latent representations generated by the EEG encoder are partly guided to consider latent representations for proprioception, while the rest are left as free latent representations. The trajectory decoder may infer actual trajectories from latent representations for proprioception and free latent representations for trajectory inference.

The proprioceptive reconstruction unit inputs electromyography, angular velocity, and acceleration data into the model, compresses and decompresses the data to create a proprioceptive latent representation that can generate data that most closely matches the original data. A loss value that measures how similar the reconstructed data (sensor signal) is to the measured data (actual sensor signal) is referred to as a 'reconstruction loss value'. The lower the reconstruction loss value $L_{recon}$, the more a latent representation inside the proprioceptive reconstruction unit is considered to effectively extract meaningful features from the measured data, and the guided latent representation guides the EEG encoder to learn sophisticated and meaningful information. That is, the reconstruction loss value may be used to guide a proprioceptive reconstruction model to extract a useful proprioceptive latent representation for trajectory generation. A criterion that evaluates the reconstruction loss value may use at least one of, for example, a mean squared error (MSE), a mean absolute percentage error (MAP), a cosine similarity, and a dynamic time warping.

The main trajectory inference unit consists of an EEG encoder and a trajectory decoder. The EEG encoder converts EEG to a latent representation, and the trajectory decoder predicts a trajectory from the latent representation. The latent representation, which is an output of the EEG encoder, is divided into a free latent representation and a joint latent representation that is guided to be similar to a proprioceptive latent representation. The joint latent representation is defined as a 'joint latent loss' $L_{latentalign}$ by calculating a similarity with the proprioceptive latent representation extracted from the proprioceptive reconstruction unit. That is, the joint latent representation loss value may be used to guide the neural encoder to extract an optimal proprioceptive latent representation from EEG. A criterion that evaluates the joint latent representation loss value may use at least one of, for example, a mean squared error (MSE), a mean absolute percentage error (MAP), a cosine similarity, and a dynamic time warping. The EEG encoder is guided to consider a proprioceptive latent representation by setting a cost function of the model in a manner of reducing the joint latent loss value.

A latent space alignment technique is used to adjust a latent space, which mainly uses a similarity between a source latent representation and a target latent representation as a loss function to guide the encoder to create the target latent representation similar to the source latent representation. Those techniques play an important role in minimizing a model loss by effectively adjusting latent representations. In this process, the model emphasizes only the meaningful characteristics of the data, allowing the artificial intelligence model to generate a more precise and personalized output. This increases the usefulness of the model in a variety of applications.

That is, a basic encoder-decoder structure is used, that takes electroencephalography as input and a trajectory of hand movement as output, and at the same time, uses a proprioceptive and free latent space optimization (PFLSO) method that guides an output latent representation of the encoder partially as a free latent representation and partially as a latent representation in consideration of proprioception, so as to allow a trajectory to be stably inferred even from complex EEG signals generated in hand movement tasks such as handwriting. In other words, the PFLSO method helps the model effectively reflect proprioceptive information from EEG signals to predict an optimal movement trajectory.

In the main trajectory inference unit, a latent vector, which is an output of the EEG encoder, is input to the trajectory decoder to finally output a trajectory. A similarity between the predicted trajectory and the actual trajectory is calculated and defined as a 'trajectory loss' $L_{trajectory}$ Using the trajectory loss value, a free latent representation of the neural encoder may be guided to extract an optimal latent representation from EEG. A criterion that evaluates the trajectory loss value may use at least one of, for example, a mean squared error (MSE), a mean absolute percentage error (MAP), a cosine similarity, and a dynamic time warping. A model cost function may be set in a manner of reducing the trajectory loss value, the entire module may be guided to take the trajectory information into account well.

The proprioceptive reconstruction unit and the main trajectory inference unit have an integrated loss value which is a composite weighted sum of three loss values, that is, a reconstruction loss value, a joint potential loss value, and a trajectory loss value, as shown in Equation 1, and the entire module may be trained simultaneously using an appropriate optimization algorithm using the integrated loss value. This is called an end-to-end learning method.

[Equation 1]

$$L_{tot} = \lambda_{re}L_{recon} + \lambda_{tr}L_{trajectory} + \lambda_{la}L_{latentalign}$$

An optimal value of each of the weights of the three loss values may be determined, for example, using a grid-search method. Additionally, each element of the module may be trained differentially over time by flexibly changing the weights according to the training level and subdividing the training steps. For example, in the early stage of training, the weight of the reconstruction loss value may be set large and the rest may be set small to intensively train the proprioceptive reconstruction unit, and from the middle stage of training, the weight of the joint latent loss value may be set large to intensively train the main trajectory inference unit.

FIG. 6 shows a process of an inference module according to one embodiment.

The inference module is a module that receives EEG as input to infer a trajectory based on the trained neural encoder and trajectory decoder. The trained main trajectory inference unit may include an EEG encoder that has been optimized through the PFLSO method, and the EEG encoder may consistently infer (predict) a hand movement trajectory in consideration of proprioceptive information by receiving only EEG signals. The trained EEG encoder takes noisy EEG as input and extracts (infers) an optimal joint latent representation (proprioceptive latent space) that has relatively less noise and is more similar to trajectory data, and an optimal free latent representation (free latent space) for trajectory generation.

In the inference module, the joint latent representation is guided to include information similar to a proprioceptive latent representation learned in the proprioceptive reconstruction unit. The joint latent representation sufficiently reflects the user's proprioceptive information to ensure consistent performance for both actual and imagined movements. The free latent representation, which is a portion that leaves the model free to learn additional information needed for trajectory prediction, helps the trajectory decoder optimally predict hand movement trajectories.

In FIG. 6, all latent vectors output from the EEG encoder are input to the trajectory decoder. Based on the latent vectors, the trajectory decoder may predict a trajectory such as an X-coordinate, a Y-coordinate, a pressure (pen pressure), a speed (force), and the like of the hand. In this process, the trajectory decoder may reflect proprioceptive information through joint latent representations and utilize additional information required for trajectory prediction through free latent representations to derive an optimal prediction result for the trajectory.

Due to consistent latent representation training using proprioception data during the training process, trajectory prediction may be performed consistently for not only actual movements but also movements imagined by the user. If a latent representation only relies on a free latent space without using the PFLSO method to infer a trajectory from EEG only with a simple encoder-decoder structure and there is no guidance of the latent representation through a separate reconstruction model, the model will only rely on noisy EEG signals, which will be less robust. If a model is trained without considering proprioceptive information, it becomes difficult to maintain consistent performance in various environments. This may lead to an unstable result in both actual and imagined hand movements, thus ultimately significantly reducing usability and usefulness. According to various embodiments of the present invention, a composite model of EEG and proprioception may be used to consistently infer a trajectory from both imagined and actually performed movements by a user.

It should be understood that various embodiments of the present invention and terms used herein are not intended to limit the technical features described to specific embodiments. With regard to the description of the drawings, similar reference numerals may be used for similar or related elements. A singular form of a noun corresponding to an item may include one or more of the things, unless the relevant context clearly indicates otherwise.

In the present disclosure, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. Terms such as "1st", "2nd", or "first" and "second" may be used merely to differentiate a corresponding element from another, and do not limit the elements in any other aspect (e.g., importance or order). When an element (e.g., a first element) is referred to as being "coupled" or "connected" to another element (e.g., a second element), with or without the term "functionally" or "communicatively," it means that the element may be connected to the other element directly (e.g., in a wired manner), in a wireless manner, or through a third element.

The term "module" as used in the present disclosure may include a unit implemented in hardware, software or firmware, and may be used interchangeably with terms such as logic, logic block, component, or circuit. A module may be an integrally configured component or a minimum unit of the component that performs one or more functions or a part thereof. For example, according to one embodiment, the module may be implemented in a form of an applicationspecific integrated circuit (ASIC).

Various embodiments may be implemented as software (e.g., a program) including one or more instructions stored in a storage medium (e.g., a memory) that is readable by a device (e.g., an electronic device). The storage medium may include a random access memory (RAM), a memory buffer, a hard drive, a database, an erasable programmable read-only memory (EPROM), an electrically erasable read-only memory (EEPROM), a read-only memory (ROM), and/or the like.

In addition, a processor in embodiments of the present invention may retrieve at least one instruction from among one or more instructions stored from a storage medium and execute the retrieved instruction. This allows the device to operate to perform at least one function according to the retrieved at least one instruction. The one or more instructions may include a code generated by a compiler or a code executable by an interpreter. The processor may be a general purpose processor, a field programmable gate array (FPGA), an application specific integrated circuit (ASIC), a digital signal processor (DSP), and/or the like.

The device-readable storage medium may be provided in a form of a non-transitory storage medium. Here, the term 'non-transitory' simply means that the storage medium is a tangible device and does not include a signal (e.g. electromagnetic waves), and this term does not differentiate between a case where data is stored semi-permanently and a case where the data is temporarily on the storage medium.

A method according to various embodiments disclosed in the present invention may be included and provided in a computer program product. The computer program product may be traded as a commodity between a seller and a buyer. The computer program product may be distributed in a form of a device-readable storage medium (e.g., compact disc read only memory (CD-ROM)), or be distributed (e.g., downloaded or uploaded) online via an application store (e.g., PlayStore) or directly between two user devices (e.g., smartphones). In the case of online distribution, at least part of the computer program product may be at least temporarily stored or temporarily generated in the device-readable storage medium, such as a manufacturer's server, a server of an application store, or a server's memory.

According to various embodiments, each element (e.g., a module, a program, a user, a target, etc.) of the above-described elements may include a single entity or a plurality of entities. According to various embodiments, one or more of the aforementioned elements or operations may be omitted, or one or more other elements or operations may be added. Alternatively or additionally, a plurality of elements may be integrated into a single element. In such a case, the integrated element may perform one or more functions of each of the plurality of elements in the same or similar manner to those performed by a corresponding one of the plurality of elements prior to the integration. According to various embodiments, operations performed by a module, a program or another element may be executed sequentially, in parallel, repeatedly, or heuristically, or one or more of the operations may be executed in a different order, omitted, or one or more other operations may be added.

## Claims

1. A method of training an artificial neural network for inferring a trajectory of an intended hand movement based on a biosignal, the method comprising:

   Collecting (205), by a first data collection part, electroencephalography data and electromyography data related to a user's hand movement;
   Collecting (210), by a second data collection part, acceleration data and angular velocity data related to the user's hand movement;
   Performing (215), by a first preprocessing part, preprocessing on each of the collected electroencephalography data, electromyography data, acceleration data, and angular velocity data;
   Extracting (220), by a proprioception data extraction part, proprioception data based on the preprocessed electromyography data, acceleration data, and angular velocity data; and
   Training (225), by a data learning part, trajectory data of the user's hand movement through an artificial neural network based on the preprocessed electroencephalography data and the extracted proprioception data.

2. The method of claim 1, wherein the training of trajectory data of the user's hand movement comprises:

   extracting proprioceptive latent representations used for data reconstruction of electromyography data, angular velocity data, and acceleration data from the extracted proprioception data;
   extracting a first free latent representation used for trajectory tracking and a first joint latent representation used for proprioceptive tracking from the preprocessed electroencephalography data; and
   determining a predicted trajectory based on the extracted proprioceptive latent representation, the first free latent representation, and the first joint latent representation.

3. The method of claim 1 or 2, wherein the data learning part determines the predicted trajectory by using a weighted integration for each of:

   a reconstruction loss value indicating a similarity between data reconstructed by the data reconstruction and actual measurement data;
   a joint latent representation loss value indicating a similarity between a first joint latent representation and a proprioceptive latent representation; and
   a trajectory loss value indicating a similarity between the predicted trajectory and an actual trajectory,
   as a loss function.

**4.** The method of claim 3, wherein each of the weights may vary over time.

**5.** The method of any one of claims 1 to 4, wherein the preprocessing on the collected electroencephalography data and electromyography data comprises filtering by a band-stop filter, filtering by a band-pass filter, independent component analysis to remove unnecessary signals, and filtering by a low-frequency band-pass filter, and
wherein the preprocessing of the angular velocity data and acceleration data comprises filtering by a correction filter.

**6.** The method of any one of claims 1 to 5, further comprising:

subsequent to performing the training of the data learning part,
collecting (230), by a third data collection part, electroencephalography data of a target related to hand movement imagination of the target;
performing (235), by a second preprocessing part, preprocessing on the collected electroencephalography data of the target; and
inferring (240), by a data inference part, trajectory data of a hand movement of the target through the artificial neural network based on the preprocessed electroencephalography data of the target.

**7.** The method of claim 6, wherein the inferring (240) of the trajectory data of the hand movement of the target comprises:

extracting, by an electroencephalography encoder, a second free latent representation used for trajectory tracking and a second joint latent representation used for proprioceptive tracking based on the preprocessed electroencephalography data input through the artificial neural network; and
inferring, by a trajectory decoder, a trajectory of hand movement of the target based on the second free latent representation and the second joint latent representation.

**8.** The method of claim 6 or 7, further comprising:
Controlling (245), by a trajectory data processing part, to transmit or display trajectory data of the inferred hand movement of the target.

**9.** A device (100) of training an artificial neural network for inferring a trajectory of an intended hand movement based on a biosignal, the device (100) comprising:

a first data collection part that collects electroencephalography data and electromyography data related to a user's hand movement;
a second data collection part that collects acceleration data and angular velocity data related to the user's hand movement;
a first preprocessing part that performs preprocessing on each of the collected electroencephalography data, electromyography data, acceleration data, and angular velocity data;
a proprioception data extraction part that extracts proprioception data based on the preprocessed electromyography data, acceleration data, and angular velocity data; and
a data learning part that trains trajectory data of the user's hand movement through an artificial neural network based on the preprocessed electroencephalography data and the extracted proprioception data.

**Patentansprüche**

**1.** Verfahren zum Trainieren eines künstlichen neuronalen Netzes zum Inferieren einer Trajektorie einer beabsichtigten Handbewegung basierend auf einem Biosignal, wobei das Verfahren umfasst:

Erfassen (205) von Elektroenzephalographiedaten und Elektromyographiedaten in Bezug auf eine Handbewegung des Benutzers durch einen ersten Datenerfassungsteil;
Erfassen (210) von Beschleunigungsdaten und Winkelgeschwindigkeitsdaten in Bezug auf die Handbewegung des Benutzers durch einen zweiten Datenerfassungsteil;
Durchführen (215) einer Vorverarbeitung der jeweils erfassten Elektroenzephalographiedaten, Elektromyographiedaten, Beschleunigungsdaten und Winkelgeschwindigkeitsdaten durch einen ersten Vorverarbeitungsteil;
Extrahieren (220) von Propriozeptionsdaten basierend auf den vorverarbeiteten Elektromyographiedaten, Beschleunigungsdaten und Winkelgeschwindigkeitsdaten durch einen Propriozeptionsdaten-Extraktionsteil; und Trainieren (225) von Trajektoriendaten der Handbewegung des Benutzers durch ein künstliches neuronales Netz basierend auf den vorverarbeiteten Elektroenzephalographiedaten und den extrahierten Propriozeptionsdaten durch einen Datenlernteil.

2. Verfahren nach Anspruch 1, wobei das Trainieren von Trajektoriendaten der Handbewegung des Benutzers umfasst:

Extrahieren propriozeptiver latenter Repräsentationen, die zur Datenrekonstruktion von Elektromyographiedaten, Winkelgeschwindigkeitsdaten und Beschleunigungsdaten verwendet werden, aus den extrahierten Propriozeptionsdaten;
Extrahieren einer ersten freien latenten Repräsentation, die zur Trajektorienverfolgung verwendet wird, und einer ersten gemeinsamen latenten Repräsentation, die zur propriozeptiven Verfolgung verwendet wird, aus den vorverarbeiteten Elektroenzephalographiedaten; und
Bestimmen einer vorhergesagten Trajektorie basierend auf der extrahierten propriozeptiven latenten Repräsentation, der ersten freien latenten Repräsentation und der ersten gemeinsamen latenten Repräsentation.

3. Verfahren nach Anspruch 1 oder 2, wobei der Datenlernteil die vorhergesagte Trajektorie unter Verwendung einer gewichteten Integration für jeden der folgenden Werte bestimmt:

einen Rekonstruktionsverlustwert, der eine Ähnlichkeit zwischen den durch die Datenrekonstruktion rekonstruierten Daten und den tatsächlichen Messdaten angibt;
einen Verlustwert der gemeinsamen latenten Repräsentation, der eine Ähnlichkeit zwischen einer ersten gemeinsamen latenten Repräsentation und einer propriozeptiven latenten Repräsentation angibt; und
einen Trajektorienverlustwert, der eine Ähnlichkeit zwischen der vorhergesagten Trajektorie und einer tatsächlichen Trajektorie angibt, als Verlustfunktion.

4. Verfahren nach Anspruch 3, wobei sich jeweils die Gewichte im Laufe der Zeit ändern können.

5. Verfahren nach einem der Ansprüche 1 bis 4, wobei die Vorverarbeitung der erfassten Elektroenzephalographiedaten und Elektromyographiedaten das Filtern durch ein Bandsperrfilter, das Filtern durch ein Bandpassfilter, eine unabhängige Komponentenanalyse zur Entfernung unnötiger Signale und das Filtern durch ein Niederfrequenz-Bandpassfilter umfasst, und
wobei die Vorverarbeitung der Winkelgeschwindigkeitsdaten und Beschleunigungsdaten das Filtern durch ein Korrekturfilter umfasst.

6. Verfahren nach einem der Ansprüche 1 bis 5, ferner umfassend:

nach Durchführung des Trainings des Datenlernteils Erfassen (230) von Elektroenzephalographiedaten eines Ziels in Bezug auf die Vorstellung einer Handbewegung des Ziels durch einen dritten Datenerfassungsteil;
Durchführen (235) einer Vorverarbeitung der erfassten Elektroenzephalographiedaten des Ziels durch einen zweiten Vorverarbeitungsteil; und
Inferieren (240) von Trajektoriendaten einer Handbewegung des Ziels durch das künstliche neuronale Netz basierend auf den vorverarbeiteten Elektroenzephalographiedaten des Ziels durch einen Dateninferenzteil.

7. Verfahren nach Anspruch 6, wobei das Inferieren (240) der Trajektoriendaten der Handbewegung des Ziels umfasst:

Extrahieren einer zweiten freien latenten Repräsentation, die zur Trajektorienverfolgung verwendet wird, und einer zweiten gemeinsamen latenten Repräsentation, die zur propriozeptiven Verfolgung verwendet wird, basierend auf den vorverarbeiteten Elektroenzephalographiedaten, die über das künstliche neuronale Netz eingegeben werden, durch einen Elektroenzephalographie-Encoder; und
Inferieren einer Trajektorie der Handbewegung des Ziels basierend auf der zweiten freien latenten Repräsentation und der zweiten gemeinsamen latenten Repräsentation durch einen Trajektorien-Decoder.

8. Verfahren nach Anspruch 6 oder 7, ferner umfassend:
Steuern (245) durch einen Trajektoriendaten-Verarbeitungsteil, um Trajektoriendaten der inferierten Handbewegung des Ziels zu übermitteln oder anzuzeigen.

9. Vorrichtung (100) zum Trainieren eines künstlichen neuronalen Netzes, um eine Trajektorie einer beabsichtigten Handbewegung basierend auf einem Biosignal zu inferieren, wobei die Vorrichtung (100) umfasst:

einen ersten Datenerfassungsteil, der Elektroenzephalographiedaten und Elektromyographiedaten in Bezug auf die Handbewegung eines Benutzers erfasst;
einen zweiten Datenerfassungsteil, der Beschleunigungsdaten und Winkelgeschwindigkeitsdaten in Bezug auf die Handbewegung des Benutzers erfasst;
einen ersten Vorverarbeitungsteil, der eine Vorverarbeitung der jeweils erfassten Elektroenzephalographiedaten, Elektromyographiedaten, Beschleunigungsdaten und Winkelgeschwindigkeitsdaten durchführt; einen Propriozeptionsdaten-Extraktionsteil, der Propriozeptionsdaten basierend auf den vorverarbeiteten Elektromyographiedaten, Beschleunigungsdaten und Winkelgeschwindigkeitsdaten extrahiert; und
einen Datenlernteil, der Trajektoriendaten der Handbewegung des Benutzers durch ein künstliches neuronales Netz basierend auf den vorverarbeiteten Elektroenzephalographiedaten und den extrahierten Propriozeptionsdaten trainiert.

## Revendications

1. Procédé d'entraînement d'un réseau neuronal artificiel pour déduire une trajectoire d'un mouvement de main prévu sur la base d'un biosignal, ledit procédé comprenant :

la collecte (205), par une première partie de collecte de données, de données d'électroencéphalographie et d'électromyographie relatives à un mouvement de main d'un utilisateur ;
la collecte (210), par une deuxième partie de collecte de données, de données d'accélération et des données de vitesse angulaire relatives au mouvement de main de l'utilisateur ;
l'exécution (215), par une première partie de prétraitement, d'un prétraitement sur les différentes données, d'électroencéphalographie, d'électromyographie, d'accélération et de vitesse angulaire collectées ;
l'extraction (220), par une partie d'extraction de données de proprioception, de données de proprioception sur la base des données d'électromyographie, des données d'accélération et des données de vitesse angulaire prétraitées ; et
l'entraînement (225), par une partie d'apprentissage de données, de données de trajectoire du mouvement de main de l'utilisateur via un réseau neuronal artificiel sur la base des données d'électroencéphalographie prétraitées et des données de proprioception extraites.

2. Procédé selon la revendication 1, où l'entraînement des données de trajectoire du mouvement de main de l'utilisateur comprend :

l'extraction de représentations latentes proprioceptives utilisées pour la reconstruction de données d'électromyographie, de vitesse angulaire et d'accélération à partir des données de proprioception extraites ;
l'extraction d'une première représentation latente libre utilisée pour un suivi de trajectoire et d'une première représentation latente conjointe utilisée pour un suivi proprioceptif à partir des données d'électroencéphalographie prétraitées ; et
la détermination d'une trajectoire prédite sur la base de la représentation latente proprioceptive extraite, de la première représentation latente libre et de la première représentation latente conjointe.

3. Procédé selon la revendication 1 ou la revendication 2, où la partie d'apprentissage de données détermine la trajectoire prédite au moyen d'une intégration pondérée pour chacune des valeurs suivantes :

une valeur de perte de reconstruction indiquant une similarité entre des données reconstruites par la reconstruction de données et des données de mesure effectives ;
une valeur de perte de représentation latente conjointe indiquant une similarité entre une première représentation latente conjointe et une représentation latente proprioceptive ; et
une valeur de perte de trajectoire indiquant une similarité entre la trajectoire prédite et une trajectoire effective, en tant que fonction de perte.

4. Procédé selon la revendication 3, où chacune des pondérations peut varier dans le temps.

5. Procédé selon l'une des revendications 1 à 4, où le prétraitement sur les données d'électroencéphalographie et d'électromyographie collectées comprend un filtrage par filtre coupe-bande, un filtrage par filtre passe-bande, une analyse de composants indépendante pour éliminer des signaux inutiles, et un filtrage par filtre passe-bas, et où le prétraitement des données de vitesse angulaire et des données d'accélération comprend un filtrage par filtre de correction.

6. Procédé selon l'une des revendications 1 à 5, comprenant en outre :

après exécution de l'entraînement de la partie d'apprentissage de données,
la collecte (230), par une troisième partie de collecte de données, de données d'électroencéphalographie d'une cible relatives à l'imagination du mouvement de main de la cible ;
l'exécution (235), par une deuxième partie de prétraitement, d'un prétraitement sur les données d'électroencéphalographie collectées de la cible ; et
la déduction (240), par une partie de déduction de données, de données de trajectoire d'un mouvement de main de la cible via le réseau neuronal artificiel sur la base des données d'électroencéphalographie prétraitées de la cible.

7. Procédé selon la revendication 6, où la déduction (240) des données de trajectoire du mouvement de main de la cible comprend :

l'extraction, par un codeur d'électroencéphalographie, d'une deuxième représentation latente libre utilisée pour un suivi de trajectoire et d'une deuxième représentation latente conjointe utilisée pour un suivi proprioceptif sur la base de l'entrée de données d'électroencéphalographie prétraitées via le réseau neuronal artificiel ; et
la déduction, par un décodeur de trajectoire, d'une trajectoire de mouvement de main de la cible sur la base de la deuxième représentation latente libre et de la deuxième représentation latente conjointe.

8. Procédé selon la revendication 6 ou la revendication 7, comprenant en outre :
la commande (245), par une partie de traitement de données de trajectoire, de la transmission ou de l'affichage des données de trajectoire du mouvement de main déduit de la cible.

9. Dispositif (100) d'entraînement d'un réseau neuronal artificiel pour déduire une trajectoire d'un mouvement de main prévu sur la base d'un biosignal, ledit dispositif (100) comprenant :

une première partie de collecte de données collectant des données d'électroencéphalographie et des données d'électromyographie relatives à un mouvement de main d'un utilisateur ;
une deuxième partie de collecte de données collectant des données d'accélération et des données de vitesse angulaire relatives au mouvement de main de l'utilisateur ;
une première partie de prétraitement exécutant un prétraitement sur les différentes données, d'électroencéphalographie, d'électromyographie, d'accélération et de vitesse angulaire collectées ;
une partie d'extraction de données de proprioception extrayant des données de proprioception sur la base des données d'électromyographie, des données d'accélération et des données de vitesse angulaire prétraitées ; et
une partie d'apprentissage de données entraînant des données de trajectoire du mouvement de main de l'utilisateur via un réseau neuronal artificiel sur la base des données d'électroencéphalographie prétraitées et des données de proprioception extraites.

Fig.1

100

Device

110
Memory

120
Processor

130
Input/output interface

140
Communication interface

Fig.2

| | |
|---|---|
| Collect electroencephalography data and electromyography data related to user's hand movement | 205 |
| Collect acceleration data and angular velocity data related to user's hand movement | 210 |
| Perform preprocessing on each of collected electroencephalography data, electromyography data, electroencephalography data, electromyography data, | 215 |
| Extract proprioception data based on preprocessed electromyography data, acceleration data, and angular velocity data | 220 |
| Train trajectory data of user's hand movement through artificial neural network based on preprocessed electroencephalography data and extracted proprioception data | 225 |
| Collect electroencephalography data of target related to hand movement imagination of target | 230 |
| Perform preprocessing on collected electroencephalography data of target | 235 |
| Infer trajectory data of hand movement of target through artificial neural network based on preprocessed electroencephalography data of target | 240 |
| Transmit or display trajectory data of inferred hand movement of target | 245 |

Fig.3

User

User interface ~340

Operating object
(cursor, virtual pen)

User interaction
(model confidence
feedback, operation
mode management)

Biosignal measurement and
transmission module
(EEG, EMG, etc.) ~310

Data preprocessing module
(ICA, Filtering, etc.) ~320

Learning mode
Yes/No

330

Neural processing unit

No                                Yes

Inference
module

Atomic
Operation

Memory

Learning
module

Database

Fig.4

```
                                          ┌─────────────────────────┐
                                          │   Electromyography/     │
                                          │ electroencephalography  │
                                          └───────────┬─────────────┘
                                                      │
                                                      ▼
                                          ┌─────────────────────────┐
                                          │     Band-stop filter    │
                                          └───────────┬─────────────┘
                                                      │
                                                      ▼
        ┌──────────────────┐             ┌─────────────────────────┐
        │    IMU sensor    │             │     Band-pass filter    │
        └──────┬────┬──────┘             └───────────┬─────────────┘
               │    │                                │
        ┌──────┘    └──────┐                         ▼
        ▼                  ▼             ┌─────────────────────────┐
  ┌─────────────┐  ┌─────────────┐       │       Independent       │
  │Angular velocity│ │ Acceleration │    │   component analysis    │
  │    signal    │  │    signal   │      └───────────┬─────────────┘
  └──────┬──────┘  └──────┬──────┘                   │
         │                │                          ▼
         └────────┬───────┘             ┌─────────────────────────┐
                  ▼                     │     Band-pass filter    │
        ┌──────────────────┐            └───────────┬─────────────┘
        │ Correction filter │                       │
        └─────────┬────────┘                        ▼
                  │                     ┌─────────────────────────┐
                  │                     │   Preprocessed biosignal │
                  │                     └───────────┬─────────────┘
                  │                                 │
                  │               ┌─────────────────┴──────────────┐
                  ▼               ▼                                ▼
  ┌───────────────────────┬──────────────────┐      ┌─────────────────────────┐
  │Preprocessed acceleration│   Preprocessed  │      │      Preprocessed       │
  │and angular velocity data│ electromyography│      │ electroencephalography  │
  └───────────┬─────────────┴────────┬────────┘      └─────────────────────────┘
              │                      │
              └──────────┬───────────┘
                         ▼
              ┌────────────────────┐
              │   Proprioception   │
              │       data         │
              └────────────────────┘
```

**EP 4 682 686 B1**

Fig.5

(4 X sensors) X times
Electromyography
(for muscle's neural activation.)
Acceleration
(for joints movements.)
Gyro [angular velocity]
(for joints movements.)
Force(for muscle tension)

Reconstruction-based model for obtaining
proprioceptive latent representation

Proprioceptive encoder → [2] → Proprioceptive decoder

(4 X sensors) X times
Electromyography
(for muscle's neural activation.)
Acceleration
(for joints movements.)
Gyro [angular velocity]
(for joints movements.)
Force(for muscle tension)

[1] [1] Margin latent representation for eeg
to trajectory (free latent)

[2] [2] Proprioceptive latent representation
(proprioceptive latent)

[3] [3] eeg to proprioceptive latent representation
(joint embedding)

EEG → EEG encoder → [1] + [3] → Trajectory decoder →
X-coordinate
Y-coordinate
Speed
Pen pressure

Input shape :
(32 EEG sensors) X times

Model that predicts trajectory from EEG
in consideration of proprioceptive information

Ouput shape :
(4D trajectory feature) X times

Fig.6

[1] Free latent representation for
trajectory decoding

[2] Proprioceptive latent
representation

EEG → EEG encoder → [1] + [3] → Trajectory decoder →
X-coordinate
Y-coordinate
Speed
Pen pressure

Input shape :
(32 EEG sensors) X times

Model that predicts trajectory from EEG
based on proprioceptive latent representation

Ouput shape :
(4D trajectory feature) X times

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020210059079 A
- KR 1020200110998 A
- KR 1020230086936 A
- KR 1020130141904 A
- US 20210018896 A1
- US 20140058528 A1

### Non-patent literature cited in the description

- An approach for upper limb movement intention recognition using EEG (electroencephalography) and sEMG (surface electromyography) fusion based on the MCPSA-CIIM (Multi-scale Convolution, Polarized Self-Attention, and Cross Intelligence Integration Module). **ZHANG WEIMING**. WRC Symposium on Advanced Robotics and Automation. IEEE, 27 September 2023, 402-407
- **AL-QURAISH MAGED S.** Multimodal fusion approach based on EEG (electroencephalography) and EMG (electromyography) signals for lower limb movement recognition. *IEEE Sensors Journal*, 14 December 2021, vol. 21 (24)
- **BALASUBRAMANIAN SIVAKUMAR**. Is EMG a Viable Alternative to BCI for Detecting Movement Intention in Severe Stroke. *IEEE Transactions on Biomedical engineering*, 20 November 2018, vol. 65 (12)
- On the feasibility of EEG-based motor intention for real-time robot assistive control. **HO JIN CHOI**. ARXIV.ORG. Cornell university library, 13 March 2024